# FASCICULE DE BREVET EUROPEEN

(11) **EP 1 190 687 B1**
(45) Date de publication et mention de la délivrance du brevet: **28.07.2004**
(21) Numéro de dépôt: 01402336.0
(22) Date de dépôt: 10.09.2001
(51) Int. Cl.: A61F 2/46

(54) **Impacteur destiné au domaine orthopédique**
Einschlaggerät zur Verwendung in der Orthopädie
Impactor for use in orthopaedics

(30) Priorité: 21.09.2000 FR 0012246
(43) Date de publication de la demande: 27.03.2002
(73) Titulaire: Sulzer Orthopedie S.A., 25461 Etupes Cédex (FR); Exaclub, 67000 Strasbourg (FR)
(72) Inventeur: Courpied, Jean-Pierre, 25000 Besancon (FR); Gibon, Yves, 25000 Besancon (FR); Leclercq, Sylvain, 25000 Besancon (FR); Moreau, Pascal, 25000 Besancon (FR); Penavayre, Jean-Luc, 25000 Besancon (FR); Vie, Pascal, 25000 Besancon (FR); Yannou, Jean-Marc, 25000 Besancon (FR); Favard, Luc, 25000 Besancon (FR); Henky, Pierre, 25000 Besancon (FR); Le Couteur, Patrick, 25000 Besancon (FR); Oget, Vincent, 25000 Besancon (FR); Slomka, Francis, 25000 Besancon (FR); Vogt, Jean-Claude, 25000 Besancon (FR); Bouraly, Jean-Pierre, 25000 Besancon (FR)
(74) Mandataire: Bentz, Jean-Paul

(56) Documents cités:
- EP-A- 0 373 078
- EP-A- 0 549 362
- WO-A-93/02642
- DE-A- 19 704 577
- US-A- 2 421 354
- US-A- 2 725 878
- US-A- 5 800 546

## Description

La présente invention concerne un impacteur destiné à produire une percussion sur un élément orthopédique à rapporter sur un autre. Le document US-A-2 421 354 représente l'état de la technique le plus proche et détermine le préambule de la revendication 1. Il s'agira notamment, mais non exclusivement d'une tête sphérique à rapporter sur une tige fémorale par l'intermédiaire d'un cône-morse réalisé de part et d'autre desdits éléments.

A l'heure actuelle ceci est obtenu par l'intermédiaire d'un outil d'impaction sur lequel le chirurgien produit des frappes selon un certain nombre et selon une certaine force.

Cette force n'est pas maîtrisée et il peut s'en suivre des inconvénients.

En effet, la tige fémorale, toujours métallique, est réalisée en titane, en chrome-cobalt ou encore en acier inoxydable et elle peut recevoir selon les cas de figures une tête sphérique métallique en chrome-cobalt ou encore en acier, mais également en céramique.

Dans ce dernier cas en particulier, si le chirurgien tape trop fort, il initialise sur la tête en céramique des fissures aboutissant ou pouvant aboutir à la rupture de cette tête ce qui est très grave, car à terme il faut réopérer le patient.

A l'inverse, qu'il s'agisse d'une tête métallique ou céramique, si la force d'impaction n'a pas été suffisante, la tête sphérique tournera sur la tige et provoquera une usure du cône-morse pour ce qui est de la tête métallique ou comme déjà énoncé ci-dessus des fissures pour la tête céramique.

En fait, pour éviter au chirurgien d'avoir à évaluer lui-même la force d'impaction nécessaire, ce qui est pratiquement impossible, étant donné les différentes formes et dimensions des cônes-morse qui varient en fonction des fabricants.

Partant de là, et selon une première phase de la démarche inventive, il a été recherché un dispositif permettant d'obtenir une force d'impaction de valeur fiable et répétitive, prédéterminée en fonction des caractéristiques géométriques du cône-morse, variables selon le fabricant.

A cet effet, l'invention concerne un impacteur destiné à produire une percussion sur un élément orthopédique à rapporter sur un autre, notamment une tête sphérique sur une tige fémorale, par l'intermédiaire d'un cône-morse, réalisé de part et d'autre desdits éléments, caractérisé en ce qu'il est constitué par un fourreau par l'intermédiaire duquel est exercée une action manuelle contre l'un des deux éléments et dans lequel fourreau est monté coulissant une masselotte de forme correspondante qui est susceptible d'agir en compression sur un ressort de face interne par l'intermédiaire de moyens de déclenchement automatique qui lui sont propres et qui sont susceptibles de se libérer à partir d'une valeur de compression prédéterminée dudit ressort, correspondant à une force d'impact de la masselotte sur l'un des éléments, définie en fonction de paramètres tels que dimensions du cône-morse et nature des matériaux le constituant.

De manière à déterminer la force du ressort de frappe, en fonction du cône-morse, il est effectué des tests de simulation au cours desquels on mesure la force minimum de frappe permettant d'avoir le maximum de force d'arrachement, correspondant au maximum de sécurité de la connexion cône-morse. C'est la raideur du ressort qui permettra d'obtenir ladite force.

Ainsi la prothèse est sécurisée car le chirurgien est certain d'avoir une connexion fiable n'offrant pas d'opportunité de mouvement d'un élément par rapport à un autre susceptible de provoquer des usures et des déconnexions.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée en référence aux dessins annexés sur lesquels :

La figure 1 représente une vue en plan d'un compacteur selon l'invention.

La figure 2 représente une vue en coupe longitudinale d'un compacteur selon la figure 1.

L'impacteur 1 désigné globalement sur les figures est particulièrement adapté à produire une percussion sur une tête sphérique d'une tige fémorale à relier entre elles par l'intermédiaire d'un cône-morse (non représenté), réalisé de part et d'autre des éléments.

Selon l'invention, l'impacteur 1 est constitué par un fourreau 2, par l'intermédiaire duquel est exercée une action manuelle F contre l'un des éléments, par exemple la rotule d'une tige fémorale, fourreau 2 dans lequel est montée coulissante une masselotte 3 de forme correspondante qui est susceptible d'agir en compression sur un ressort de frappe interne 4 par l'intermédiaire de moyens de déclenchement automatique 5 qui lui sont propres, se libérant à partir d'une valeur de compression prédéterminée dudit ressort 4, correspondant à une force d'impact de la masselotte 3 sur l'un des éléments, en l'occurrence une rotule.

Cette force d'impact est définie en fonction de paramètre tels que dimensions du cône-morse et nature des matériaux le constituant.

C'est ainsi que cette force d'impact pourra être modulée en fonction de ces paramètres, en interchangeant le ressort de frappe 4.

Une campagne d'essais a été menée afin de tester des têtes en zircone de 22,2 mm avec des cônes de huit dixièmes. Une courbe a été définie, corrélant la charge d'impaction à l'effort de désimpaction. Cette courbe est logarithmique. Au-delà de 1500 daN, il y a déformation plastique.

A 1000 daN, on caractérise dans les tests pull-in/pull-out, la nature des cône-morse. La charge appliquée par un chirurgien prudent en impactant la tête est très inférieure à cette valeur. Afin d'avoir un coefficient de sécurité suffisant et de ne prendre aucun risque d'ébranler le ciment, un test consiste à évaluer sur bois tendre l'empreinte réalisée par l'impaction de la tête sur le cône par des coups modérés. La charge appliquée par l'impacteur selon l'invention doit donner la même empreinte.

La technique opératoire consiste à impacter d'abord la tige fémorale. La longueur du col ayant été évaluée, la tête sphérique est placée sur la tige. L'impacteur à percussion selon l'invention est ensuite positionné sur la tête sphérique et mis en appui manuellement jusqu'au déclenchement de la masselotte.

Selon le mode de réalisation, cité à titre d'exemple sur les figures, les moyens de déclenchement 5 sont constitués par un loquet 6 monté en bascule sur un axe d'articulation 7 excentré, monté solidairement sur les parois d'un logement 8 de la masselotte 3 dans lequel il est logé.

Un tirant axial 9 traverse librement un alésage 10 de la masselotte 3 mais est susceptible d'être solidarisé de celle-ci par le loquet 6 pour être entraîné vers le ressort de frappe 4 disposé entre un fond 2a du fourreau 2 et la masselotte 3, de manière à le comprimer jusqu'à une valeur de déclenchement à partir de laquelle le loquet 6 bascule et libère la masselotte 3 par rapport au tirant 9.

Celle-ci est alors projetée en force, dans une direction opposée à celle de compression du ressort 4, pour venir frapper un embout d'impaction 11 traversant librement l'autre fond 2b du fourreau 2 opposé au ressort 4 et solidaire d'une extrémité 9a du tirant 9 débouchant dudit fond 2b du fourreau 2, lequel embout 11 est maintenu en application par l'opérateur sur l'un des éléments à assembler.

En l'occurrence, l'embout 11 est sphérique pour s'adapter, selon le présent exemple de réalisation, sur une tête sphérique d'une tige fémorale.

Plus précisément, le loquet est constitué par une pièce globalement cylindrique apte d'une part à se loger avec jeu dans le logement 8, sensiblement correspondant, ménagé à la partie supérieure de la masselotte 3 vers le ressort de frappe 4 et à y être articulé en bascule autour de l'axe 7 solidaire de ladite masselotte 3 s'étendant perpendiculairement à l'axe longitudinal de l'ensemble, mais décalé par rapport à celui-ci, et d'autre part à être traversé librement par le tirant 9 de la même manière que la masselotte 3 avec lequel est susceptible d'être solidarisé ladite masselotte 3 et son loquet 6 par l'intermédiaire d'une dent 12 réalisée à la partie inférieure de ce dernier et dirigée vers l'axe longitudinal du tirant 9 pour coopérer en enclenchement avec une gorge périphérique 13 de celui-ci sous l'influence d'un ressort 14 interposé entre une paroi du logement supérieur 8 de la masselotte 3 et le loquet 6 au droit de sa dent 12.

Celle-ci demeure enclenchée dans la gorge 13 du tirant 9 au cours d'une poussée en compression sur le ressort de frappe 4 exercée par l'embout d'impaction 11 solidaire du tirant 9, lui-même solidaire temporairement de la masselotte 3, par l'intermédiaire du loquet 6 jusqu'à provoquement du basculement de celui-ci autour de son axe 7, s'effectuant par poussée sur le loquet 6 à sa partie supérieure diagonalement opposée à la dent 12, par l'intermédiaire d'un appui 15 venant en contact avec une partie fixe interne 16a d'un bouchon de fermeture 16 du fourreau 2, pour provoquer le déclenchement du loquet 6 d'où de la masselotte 3 qui en est solidaire.

Le bouchon de fermeture 16 du fourreau 2 est constitué par une partie supérieure 16b en forme de couvercle se vissant dans une partie correspondante filetée du fourreau 2 et par une partie interne 16c délimitant d'une part un logement axial borgne 17 permettant le libre débattement du tirant 9 et d'autre part un logement coaxial 18, entre ladite partie interne 16c du bouchon 16 et l'alésage 2c du fourreau 2.

Dans ce logement coaxial 2c est disposé le ressort de frappe 4, entre le sommet périphérique 3a de la masselotte 3 et un épaulement 16d formé par le dessous de la partie de bouchon 16 formant couvercle 16b.

Selon une autre caractéristique de l'invention un ressort de rappel 19, de force inférieure à celle du ressort de frappe 4, est disposé coaxialement à une partie inférieure du tirant 9 entre un logement interne 20 de l'embout d'impaction 11 et un logement interne 21 de la masselotte 3.

Enfin, selon une autre caractéristique de l'invention le fourreau 2 comporte sur sa périphérie des ouïes 22 traversant sa paroi de manière à permettre une stérilisation des parties externes et internes de l'ensemble.

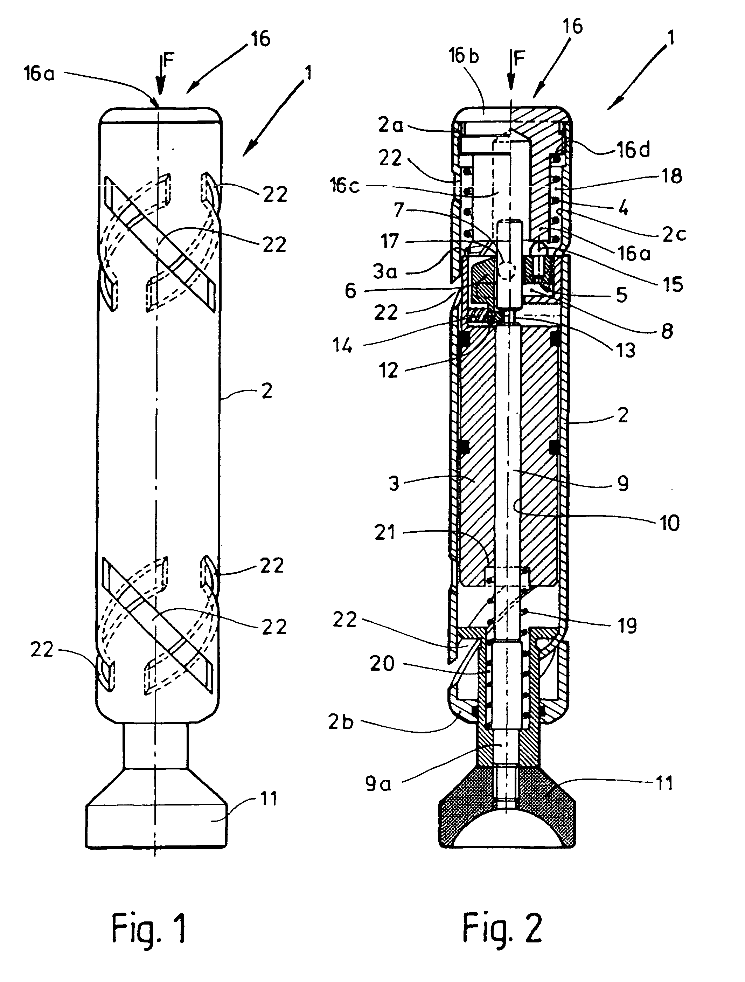

L'impacteur représenté sur la figure 2 correspond à une position immédiatement avant déclenchement.

En résumé, le fonctionnement de l'impacteur est le suivant.

Lorsque l'on exerce une poussée F sur le fourreau 2 ou encore sur sa partie d'extrémité formée par le bouchon 16, on provoque une poussée correspondante sur l'embout d'impaction 11 qui étant solidaire du tirant 9 pousse celui-ci qui entraîne conséquemment la masselotte 3 par l'intermédiaire du loquet 6 pour entraîner l'ensemble vers le ressort de frappe 4 en butée sous le bouchon 16 qui est fixe.

Cette compression s'exerce jusqu'à ce que l'appui 15 du loquet basculant 6 vienne en contact avec le bouchon 16.

A ce moment là, le basculement du loquet 6 provoque le désengagement de sa dent 12 de la gorge 13 du tirant 9, ce qui provoque le retour violent vers le bas de la masselotte 3, pour percuter l'embout 11 et provoquer une impaction dont la valeur est en fonction des caractéristiques du ressort de frappe 4.

## Revendications

1. Impacteur destiné à produire une percussion sur un élément orthopédique à rapporter sur un autre, notamment une tête sphérique sur une tige fémorale, par l'intermédiaire d'un cône-morse, réalisé de part et d'autre desdits éléments, lequel impacteur est constitué par un fourreau (2) dans lequel est monté coulissant une masselotte (3) qui est susceptible d'agir en compression sur un ressort (4) par l'intermédiaire de moyens de déclenchement automatique (5), **caractérisé en ce que** les moyens de déclenchement (5) sont constitués par un loquet (6) monté en bascule sur un axe d'articulation (7) excentré, monté solidairement sur les parois d'un logement (8) de la masselotte (3) dans lequel il est logé, un tirant axial (9) traversant librement un alésage (10) de la masselotte (3) mais susceptible d'être solidarisé de celle-ci par le loquet (6) pour être entraîné vers le ressort de frappe (4) disposé entre un fond (2a) du fourreau (2) et la masselotte (3), de manière à le comprimer jusqu'à une valeur de déclenchement à partir de laquelle le loquet (6) bascule et libère la masselotte (3) par rapport au tirant (9), celle-ci étant alors projetée en force, dans une direction opposée à celle de compression du ressort (4), pour venir frapper un embout d'impaction (11) traversant librement l'autre fond (2b) du fourreau (2) opposé au ressort (4) et solidaire d'une extrémité (9a) du tirant (9) débouchant dudit fond (2b) du fourreau (2), lequel embout (11) est maintenu en application par l'opérateur sur l'un des éléments à assembler.

2. Impacteur selon la revendication 1, **caractérisé en ce que** le loquet (6) est constitué par une pièce globalement cylindrique apte d'une part à se loger avec jeu dans le logement (8), sensiblement correspondant, ménagé à la partie supérieure de la masselotte (3) vers le ressort de frappe (4) et à y être articulé en bascule autour de l'axe (7) solidaire de ladite masselotte (3) s'étendant perpendiculairement à l'axe longitudinal de l'ensemble, mais décalé par rapport à celui-ci, et d'autre part à être traversé librement par le tirant (9) de la même manière que la masselotte (3) avec lequel est susceptible d'être solidarisé ladite masselotte (3) et son loquet (6) par l'intermédiaire d'une dent (12) réalisée à la partie inférieure de ce dernier et dirigée vers l'axe longitudinal du tirant (9) pour coopérer en enclenchement avec une gorge périphérique (13) de celui-ci sous l'influence d'un ressort (14) interposé entre une paroi du logement supérieur (8) de la masselotte (3) et le loquet (6) au droit de sa dent (12), celle-ci demeurant enclenchée dans la gorge (13) du tirant (9) au cours d'une poussée en compression sur le ressort de frappe (4) exercée par l'embout d'impaction (11) solidaire du tirant (9), lui-même solidaire temporairement de la masselotte (3), par l'intermédiaire du loquet (6) jusqu'à provoquement du basculement de celui-ci autour de son axe (7), s'effectuant par poussée sur le loquet (6) à sa partie supérieure diagonalement opposée à la dent (12), par l'intermédiaire d'un appui (15) venant en contact avec une partie fixe interne (16a) d'un bouchon de fermeture (16) du fourreau (2), pour provoquer le déclenchement du loquet (6) d'où de la masselotte (3) qui en est solidaire.

3. Impacteur selon la revendication 2, **caractérisé en ce que** le bouchon de fermeture (16) du fourreau (2) est constitué par une partie supérieure (16b) en forme de couvercle se vissant dans une partie correspondante filetée du fourreau (2) et par une partie interne (16c) délimitant d'une part un logement axial borgne (17) permettant le libre débattement du tirant (9) et d'autre part un logement coaxial (18), entre ladite partie interne (16c) du bouchon (16) et l'alésage (2c) du fourreau (2), logement coaxial (2c) dans lequel est disposé le ressort de frappe (4), entre le sommet périphérique (3a) de la masselotte (3) et un épaulement (16d) formé par le dessous de la partie de bouchon (16) formant couvercle (16b).

4. Impacteur selon l'une des revendications 1 à 3, **caractérisé en ce qu'**un ressort de rappel (19), de force inférieure à celle du ressort de frappe (4), est disposé coaxialement à une partie inférieure du tirant (9) entre un logement interne (20) de l'embout d'impaction (11) et un logement interne (21) de la masselotte (3).

5. Impacteur selon l'une des revendications 1 à 4, **caractérisé en ce que** le fourreau (2) comporte sur sa périphérie des ouïes (22) traversant sa paroi de manière à permettre une stérilisation des parties externes et internes de l'ensemble.

## Patentansprüche

1. Stoßkörper, mit dem ein Schlag auf ein orthopädisches Element, das an ein anderes mittels eines Morsekonus, der beidseitig der Elemente realisiert ist, angefügt werden soll, und insbesondere auf einen sphärischen Kopf an einem femoralen Schaft, ausgeführt werden kann, wobei der Stoßkörper durch eine Scheide (2) gebildet ist, in der gleitend ein Reguliergewicht (3) montiert ist, das unter Druck mit Hilfe automatischer Auslösemittel (5) auf eine Feder (4) wirken kann, **dadurch gekennzeichnet, daß** die Auslösemittel (5) durch ein Schnappschloß (6), das schwenkbar an einer exzentrischen Anlenkachse (7) montiert ist, die fest an den Wänden einer Aufnahme (8) des Reguliergewichts (3) montiert ist, in der das Schnappschloß (6) aufgenommen ist, und einen axialen Stab (9) gebildet werden, der frei eine Bohrung (10) des Reguliergewichts (3) durchsetzt, jedoch fest mit dem Reguliergewicht (9) durch das Schnappschloss (6) verbunden sein kann, so daß der Stab (9) in Richtung der Schlagfeder (4) mitgenommen wird, die zwischen einem Boden (2a) der Scheide (2) und dem Reguliergewicht (3) so angeordnet ist, daß die Schlagfeder (4) bis zu einem Auslösewert komprimiert wird, ausgehend von dem das Schnappschloß (6) schwenkt und das Reguliergewicht (3) im Verhältnis zum Stab (9) freigibt, wobei das Reguliergewicht (9) somit mit Schwung in einer Richtung entgegengesetzt zur Richtung der Kompression der Feder (4) geschleudert wird, um auf einen Anschlag (11) aufzutreffen, der frei den anderen Boden (2b) der Scheide (2) gegenüberliegend der Feder (4) durchquert und mit einem Ende (9a) des Stabes (9), der aus dem Boden (2b) der Scheide (2) mündet, fest verbunden ist, wobei der Anschlag (11) durch den Operierenden in Anlage an einem der zusammenzufügenden Elemente gehalten wird.

2. Stoßkörper nach Anspruch 1, **dadurch gekennzeichnet, daß** das Schnappschloß (6) durch ein im allgemeinen zylindrisches Stück gebildet wird, das so ausgebildet ist, daß das Schnappschloß (6) einerseits mit Spiel in der Aufnahme (8) aufgenommen ist, die im wesentlichen entsprechend am oberen Teil des Reguliergewichts (3) in Richtung der Schlagfeder (4) installiert ist, und dort schwenkbar um die Achse (7) angelenkt ist, die fest mit dem Reguliergewicht (3) verbunden ist und sich senkrecht zur longitudinalen Achse der Gesamtheit erstreckt, jedoch im Verhältnis zu dieser versetzt ist, und andererseits frei durch den Stab (9) in derselben Weise wie das Reguliergewicht (3) durchsetzt ist, wobei das Reguliergewicht (3) und sein Schnappschloß (6) so ausgebildet sind, daß sie mit dem Stab (9) mittels eines Zahns (12) verbunden sind, der am unteren Teil des Schnappschlosses (6) ausgebildet ist und in Richtung zur longitudinalen Achse des Stabs (9) gerichtet ist, um durch Einrasten mit einer peripheren Rille (13) des Stabes (9) unter der Einwirkung einer Feder (14) zusammenzuwirken, die zwischen eine Wand der darüberliegenden Aufnahme (8) des Reguliergewichts (3) und das Schnappschloß (6) in geradliniger Ausrichtung mit dessen Zahn (12) eingefügt ist, der in der Rille (13) des Stabes (9) bei Ausübung einer Längskraft zur Kompression der Schlagfeder (4) eingerastet bleibt, wobei die Längskraft durch den mit dem Stab (9) fest verbundenen Anschlag (11) ausgeübt wird und der Stab (9) seinerseits temporär mit dem Reguliergewicht (3) mittels des Schnappschlosses (6) fest verbunden ist, bis das Schwenken desselben um seine Achse (7) durch Drücken auf das Schnappschloß (6) an seinem oberen Teil diagonal gegenüberliegend zum Zahn (12) mittels eines Anschlags (15) bewirkt wird, wobei der Anschlag (15) in Kontakt mit einem inneren festen Teil (16a) eines Verschlußstopfens (16) der Scheide (2) gelangt und das Schnappschloß (6) und das Reguliergewicht (3), das mit diesem fest verbunden ist, auslöst.

3. Stoßkörper nach Anspruch 2, **dadurch gekennzeichnet, daß** der Verschlußstopfen (16) der Scheide (2) durch einen oberen Teil (16b) in Form eines Deckels, der auf einen entsprechenden mit einem Gewinde versehenen Teil der Scheide (2) geschraubt wird, und durch einen inneren Teil (16c) gebildet ist, der einerseits eine blinde axiale Aufnahme (17), die das freie Ausschlagen des Stabes (9) ermöglicht, und andererseits eine koaxiale Aufnahme (18) zwischen dem inneren Teil (16c) des Stopfens (16) und der Bohrung (2c) der Scheide (2) abgrenzt, wobei eine koaxiale Aufnahme (2c) gebildet wird, in der die Schlagfeder (4) zwischen dem peripheren Scheitel (3a) des Reguliergewichts (3) und einer Schulter (16d) angeordnet ist, die durch den unteren Teil des Stopfenteils (16), der den Deckel (16b) bildet, gebildet wird.

4. Stoßkörper nach einem der Ansprüche 1 bis 3, **dadurch gekennzeichnet, daß** eine Rückholfeder (19) mit geringerer Kraft als der der Schlagfeder (4) koaxial an einem unteren Teil des Stabes (9) zwischen einer inneren Aufnahme (20) des Anschlages (11) und einer inneren Aufnahme (21) des Reguliergewichts (3) angeordnet ist.

5. Stoßkörper nach einem der Ansprüche 1 bis 4, **dadurch gekennzeichnet, daß** die Scheide (2) auf ihrer Peripherie Löcher (22) aufweist, die ihre Wand derart durchsetzen, daß eine Sterilisation der äußeren und inneren Teile der Gesamtheit möglich ist.

## Claims

1. Impactor intended to produce a percussion on an orthopaedic element to be attached to another, in particular a spherical head to be attached to a femoral stem, by way of a Morse cone formed on each side of said elements, which impactor consists of a sleeve (2) in which a weight (3) is mounted slidably, which weight (3) is able to act with compression on a spring (4) by way of automatic release means (5), **characterized in that** the release means (5) consist of a catch (6) mounted with tilting on an eccentric hinge pin (7) which is mounted integrally on the walls of a seat (8) of the weight (3) in which it is lodged, an axial tie rod (9) passing freely through a bore (10) of the weight (3) but being able to be made integral with the latter by the catch (6) so as to be driven towards the striker spring (4) arranged between one end (2a) of the sleeve (2) and the weight (3), in such a way as to compress it as far as a release value at which the catch (6) tilts and frees the weight (3) relative to the tie rod (9), this weight then being projected with force, in a direction counter to that of compression of the spring (4), in order to strike an impaction endpiece (11) which passes freely through the other end (2b) of the sleeve (2) remote from the spring (4) and is integral with an end (9a) of the tie rod (9) opening out from said end (2b) of the sleeve (2), the operator keeping the endpiece (11) applied against one of the elements to be joined together.

2. Impactor according to Claim 1, **characterized in that** the catch (6) consists of a generally cylindrical component which is able, on the one hand, to fit with play in the substantially corresponding seat (8) formed at the upper part of the weight (3) towards the striker spring (4) and to be articulated there with tilting around the pin (7) which is integral with said weight (3) and extends perpendicular to the longitudinal axis of the assembly, but offset relative to the latter, and, on the other hand, to be traversed freely by the tie rod (9), in the same way as the weight (3), and with which it is possible to connect said weight (3) and its catch (6) by way of a tooth (12) formed at the lower part of the latter and directed towards the longitudinal axis of the tie rod (9) so as to cooperate by locking with a peripheral groove (13) of the latter under the effect of a spring (14) interposed between a wall of the upper seat (8) of the weight (3) and the catch (6) in line with its tooth (12), the latter remaining locked in the groove (13) of the tie rod (9) during a compression thrust on the striker spring (4) exerted by the impaction endpiece (11) integral with the tie rod (9), itself temporarily integral with the weight (3) by way of the catch (6) until the latter is caused to tilt about its pin (7), effected by a thrust on the catch (6) at its upper part diagonally opposite the tooth (12), by way of a bearing (15) coming into contact with an internal fixed part (16a) of a closure plug (16) of the sleeve (2), so as to provoke the release of the catch (6) and, consequently, of the weight (3) which is integral with it.

3. Impactor according to Claim 2, **characterized in that** the closure plug (16) of the sleeve (2) consists of an upper part (16b) in the form of a lid screwed into a corresponding threaded part of the sleeve (2), and of an internal part (16c) delimiting, on the one hand, a blind axial seat (17) permitting free clearance of the tie rod (9), and, on the other hand, a coaxial seat (18) between said internal part (16c) of the plug (16) and the bore (2c) of the sleeve (2), in which coaxial seat (2c) is arranged the striker spring (4), between the peripheral summit (3a) of the weight (3) and a shoulder (16d) formed by the bottom of the part of the plug (16) forming a lid (16b).

4. Impactor according to one of Claims 1 to 3, **characterized in that** a return spring (19), with a force less than that of the striker spring (4), is arranged coaxially at a lower part of the tie rod (9) between an internal seat (20) of the impaction endpiece (11) and an internal seat (21) of the weight (3).

5. Impactor according to one of Claims 1 to 4, **characterized in that** the sleeve (2) comprises, on its periphery, slits (22) extending through its wall so as to permit sterilization of the external and internal parts of the assembly.
